# EUROPEAN PATENT SPECIFICATION

(11) **EP 2 238 914 B1**
(45) Date of publication and mention of the grant of the patent: **20.05.2015**
(21) Application number: 10159241.8
(22) Date of filing: 07.04.2010
(51) Int. Cl.: A61B 17/04

(54) **Twist-in suture anchor**
Eingedrehter Nahtanker
Ancrage de suture par torsion

(30) Priority: 10.04.2009 US 168520 P
(43) Date of publication of application: 13.10.2010
(73) Proprietor: Arthrex, Inc., Naples, FL 34108-1945 (US)
(72) Inventor: Shuler, Donald K., Naples, FL 34110 (US); Benavitz, William C., Naples, FL 34119 (US); Dooney, Jr., Thomas, Naples, FL 34119 (US); Sodeika, John A., Naples, FL 34119 (US)
(74) Representative: Ehrner & Delmar Patentbyrå AB

(56) References cited:
- WO-A2-02/09601
- US-A- 5 370 662
- US-A- 5 607 432
- US-A1- 2007 142 837
- US-A1- 2008 306 510

## Description

### BACKGROUND OF THE INVENTION

### 1. Field of the Invention:

The present invention relates to suture anchors used for attachment of tissue to bone and, more particularly, to a suture anchor that is provided with threads and that is pounded into a pilot hole in bone without requiring tapping of the hole or turning of the driver handle.

### 2. Description of the Related Art:

Various types of suture anchors have been developed for reattachment of soft tissue to bone. Some suture anchors are designed to be inserted into a pre-drilled hole (by twisting the anchor employing a suture anchor driver, for example), while some suture anchors are self-tapping. For example, U.S. Pat. No. 4,632,100 discloses a self-drilling, self-tapping cylindrical suture anchor which includes a drill bit at a leading end for boring a hole in a bone. The drill bit at the leading end is followed by a flight of threads for securing the anchor into the hole bored in the bone by the drill bit. Another example is U.S. Pat. No. 5,370,662 which discloses a self-tapping suture anchor having a flight of threads around a solid body. Similarly, U.S. Pat. No. 5,156,616 discloses a suture anchor having an axial opening for holding a knotted piece of suture.

US publications No. 2008/0306510 A1 and 2007/0142837, refer to suture anchors described as being rotated clockwise or counterclockwise by a surgeon, corresponding to the preamble of claim 1.

US patent 5,607,432 teaches a suture anchor retriever for a suture anchor adapted for engaging a drive portion of a threaded anchor and for turning it in a direction to remove the anchor from the implantation site.

A suture anchor that is provided with threads and that is inserted within a pilot hole without requiring tapping of the hole and without requiring turning or twisting the driver handle is needed. A suture anchor that is designed to twist by itself upon insertion into a drilled pilot hole, by only malleting the handle of a driver, is also needed.

### SUMMARY OF THE INVENTION

The present invention provides a pound-in suture anchor that self-twists upon insertion into a drilled pilot hole. The suture anchor of the present invention is provided with threads that allow the anchor to be inserted within a pilot hole without tapping the pilot hole and without turning (or twisting) the driver handle. The suture anchor of the present invention is designed to twist by itself upon insertion into the drilled pilot hole, while malleting the driver handle. Insertion of the suture anchor within the drilled pilot hole does not require the surgeon to physically twist the anchor, as with the prior art suture anchors.

The pound-in or twist-in suture anchor of the present invention is secured easily and effectively, especially in softer types of bone, without displacing a great amount of bone upon insertion. The pound-in or twist-in suture anchor of the present invention also has increased pull-out strength and prevents "backing out" of the insertion site.

These and other features and advantages of the invention will be more apparent from the following detailed description that is provided in connection with the accompanying drawings and illustrated exemplary embodiments of the invention.

### BRIEF DESCRIPTION OF THE DRAWINGS

FIG. 1 illustrates a side view of a suture anchor according to a first embodiment of the present invention, and provided with a quad lead and a 2-thread pitch.

FIG. 2 illustrates a side view of a suture anchor according to a second embodiment of the present invention, and provided with a quad lead and a 1-thread pitch.

FIG. 3 illustrates the suture anchor of FIG. 2 before insertion into a drilled pilot hole.

FIG. 3(a) illustrates the suture anchor of FIG. 3 after insertion into the drilled pilot hole (rotated, by itself, at about 100 degrees on full insertion).

FIG. 4 illustrates the suture anchor of FIG. 1 (2-thread pitch) before insertion into a drilled pilot hole.

FIG. 4(a) illustrates the suture anchor of FIG. 4 after insertion into the drilled pilot hole (rotated, by itself, at about 60 degrees on full insertion).

FIG. 5 illustrates a perspective view of a suture anchor according to a third embodiment of the present invention.

FIG. 5(a) illustrates another perspective view of the suture anchor of FIG. 5.

FIG. 6 illustrates the suture anchor of FIG. 5 before insertion into a drilled pilot hole.

FIG. 6(a) illustrates the suture anchor of FIG. 6 after insertion into the drilled pilot hole (rotated, by itself, at more than about 90 degrees on full insertion).

FIG. 7 illustrates a side view of a suture anchor assembly including a suture anchor, threaded with suture, and a driver according to the present invention.

### DETAILED DESCRIPTION OF THE PREFERRED EMBODIMENTS

The following description is provided to enable any person skilled in the art to make and use the invention and sets forth the best modes contemplated by the inventors of carrying out their invention. Various modifications, however, will remain readily apparent to those skilled in the art.

The present invention provides a pound-in suture anchor that twists upon insertion into a drilled pilot hole. The suture anchor of the present invention is provided with threads that allow the suture to be inserted within a pilot hole without tapping the hole and without turning (or twisting) the driver handle. The suture anchor of the present invention is designed to twist by itself upon insertion into the drilled pilot hole, upon malleting of the driver handle. Insertion of the suture anchor within the drilled pilot hole does not require the surgeon to physically twist the anchor, as with the prior art suture anchors.

The pound-in or twist-in suture anchor of the present invention is secured easily and effectively, especially in softer types of bone, without displacing a great amount of bone upon insertion. The pound-in or twist-in suture anchor of the present invention also has increased pull-out strength and prevents "backing out" of the insertion site.

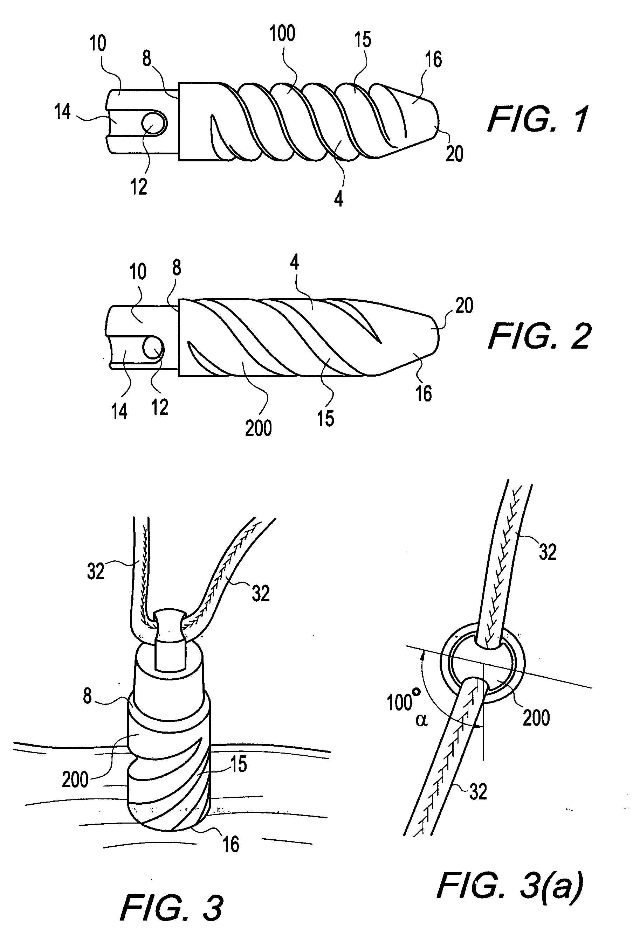

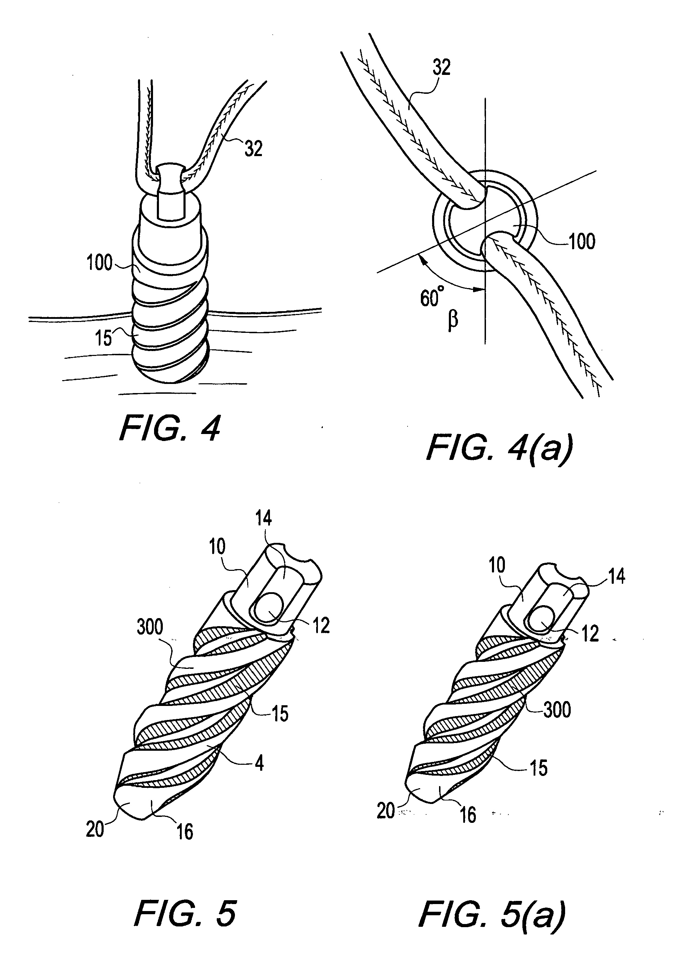

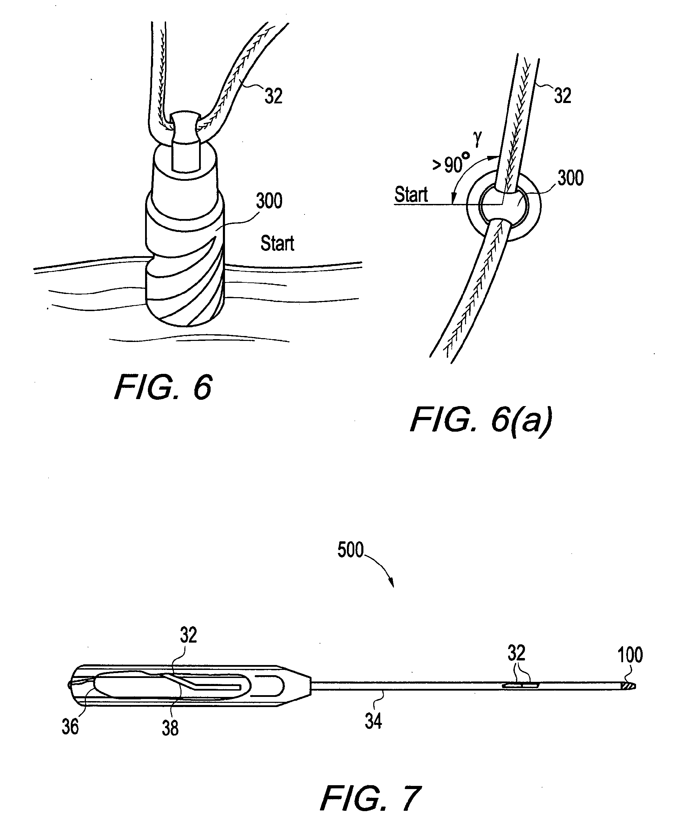

Referring now to the drawings, where like elements are designated by like reference numerals, Figures 1-6 illustrate various views of twist-in suture anchors 100, 200, 300 of the present invention. Figure 7 illustrates a side view of a suture anchor assembly 500 including a suture anchor 100, 200, 300 (threaded with suture 32) and a driver 34 according to the present invention. Preferably, the twist-in suture anchors 100, 200, 300 of the present invention are formed of PEEK or other bioabsorbable material.

Figure 1 illustrates an exemplary embodiment of suture anchor 100 provided with a quad lead and a 2-thread pitch, while Figure 2 illustrates another exemplary embodiment of suture anchor 200 provided with a quad lead and a 1-thread pitch. Figure 3 illustrates a third embodiment of suture anchor 300 with a more aggressive thread compared to that of suture anchors 100, 200.

In an exemplary embodiment only, suture anchors 100, 200, 300 are provided with four large helical threads 15 which cause the anchors to twist by themselves upon insertion in drilled pilot holes. Proximal end 8 of body 4 of suture anchor 100, 200, 300 is provided with a drive head 10 having a suture eyelet 12. The suture eyelet 12 may preferably be in the form of an oval or circular aperture located in the drive head, for holding one or more flexible suture strands 32 (for example, braided, high strength suture, sold under the tradename FiberWire®). Channels 14 may be formed along either side of the drive head 10 to accommodate the one or multiple suture strands (when the suture anchor is held in a suture anchor driver). Tip 20 of suture anchors 100, 200, 300 may terminate in a rounded point or may be blunt. The helical threads 15 are cut within the body of the suture anchors 100, 200, 300 and wrap around the whole length of the anchor body (i.e., extending the whole length of body 4 and also on the whole length of tip 20). The anchors of the present invention have a preferred pitch angle of between 20° to 70°.

Body 4 of suture anchor 100, 200, 300 may preferably be formed of a non-resilient biocompatible material such a bioabsorbable material, poly(1-lactide-co-d,1-lactide) 70:30 (PLDLA) being most preferred. Body 4 may be also formed of PEEK (for example, industrial grade PEEK), stainless steel or titanium alloy, among others. The body preferably is circular in cross-section, and tapers from a maximum diameter near proximal end 8 to a minimum diameter toward distal end 16. In a preferred embodiment, however, the body 4 has a constant diameter while the tip 20 is provided with a tapered, conical shape and a tip diameter that ranges from the constant diameter of the body 4 to a minimum diameter at a most distal end of the tip 20.

Suture anchors 100, 200, 300 of the present invention are not provided with any barbs and are non-flexible. The anchors rotate or twist as they are advanced, without requiring the surgeon to physically tap a pilot hole, rotate the anchors, or pull back to seat the anchors. The large helical threads of anchors 100, 200, 300 allow these anchors to twist upon insertion by just malleting the handle of the suture anchor driver. Tests performed on suture anchors 100, 200, 300 of the present invention demonstrate that the anchors rotate about 60 to about 100 degrees on insertion (as shown by angles α, β and γ in Figures 3, 4 and 6, respectively).

The suture anchor 100, 200, 300 is inserted into a hole formed in bone. The hole can be formed by punching or boring, for example. The helical threads secure the anchor in the bone. Advantageously, the hole formed in bone is made deep enough, and the suture anchor 100, 200, 300 is advanced into the hole sufficiently, so that the proximal end of the anchor sits flush with or below the bone surface. Accordingly, the repair leaves a smooth bone surface, minimizing or eliminating abrasion or other damage to surrounding soft tissue. The anchor generally becomes encapsulated by fibrous tissue within six weeks after implantation.

Figure 7 illustrates a suture anchor assembly 500 comprising a twist-in suture anchor 100, 200, 300 of the present invention secured on a driver 34. The anchors are designed such that there is a smooth, round interface between the driver and implant to allow rotation upon insertion into bone. In an exemplary embodiment, suture anchor 100 threaded with two pieces of suture 32 is held on the cannulated suture anchor driver 34. The suture is wrapped around cleat 36. Tension on the suture aids in retaining the suture anchor in the distal end of the driver. If desired, the suture is held in place in slot 38 using a foam adhesive, for example, during shipping. The assembly may be provided as a sterile unit ready for surgical application. The anchor 100 is provided with four large helical threads which cause the anchor to twist upon insertion while malleting the driver handle.

A pilot hole is prepared in bone using either a punch or a drill depending on surgeon's preference. At least one strand of suture 32 is loaded through the eyelet of suture anchor 100, 200, 300. The suture anchor is positioned on a cannulated driver (such as driver 34) so that the most distal end of the driver securely engages the drive head 10 provided with suture eyelet 12 and the end of the driver abuts against the threaded body of the anchor. The suture anchor with driver is inserted into the prepared pilot hole by hand. The suture anchor/implant is secure to the driver but free to rotate. A mallet is used to advance the suture anchor 100, 200, 300 into the hole. The anchor is advanced by twisting upon insertion, as a result of malleting the handle of the driver.

The suture anchor 100, 200, 300 of the present invention is particularly well suited for reattachment of the glenoid labrum or inferior glenohumeral ligament in patients with primary or recurrent anterior dislocation or subluxation of the shoulder in association with adequate post-operative immobilization. More specifically, the anchor also can be used for repair procedures such as capsulabral plication.

Although the present invention has been described in connection with preferred embodiments, many modifications and variations will become apparent to those skilled in the art. While preferred embodiments of the invention have been described and illustrated above, it should be understood that these are exemplary of the invention and are not to be considered as limiting.

## Claims

1. A pound-in suture anchor (100, 200, 300), comprising:
a non-cannulated central body (4) having a distal end (16) and a proximal end (8);
a tip portion (20) located at the distal end (16) of the central body (4), the tip portion (20) having a tapered, conical shape terminating in a rounded point or being blunt;
a drive head (10) formed on the proximal end (8) of the central body (4), the drive head (10) being provided with two channels formed along sides of the drive head (10) to accommodate at least one strand of suture (32);
an eyelet (12) for receiving the at least one strand of suture (32) formed on the proximal end (8) of the central body (4); and **characterised in that** the central body (4) has a constant diameter and **in that** a continuous helical thread comprises a quad lead with four helical threads and a 1-thread pitch or a 2-thread pitch, wherein the helical threads are disposed in a spiral around a whole length of the central body (4) and around a whole length of the tip portion (20), the continuous helical thread being configured to cause the anchor (100, 200, 300) to self-rotate by itself about 60 to about 100 degrees upon insertion into a hole in bone by only malleting a handle of a driver (34) engaging the drive head of the anchor (100, 200, 300) and pounding the driver (34) and the anchor (100, 200, 300) into the hole in bone, and without requiring tapping of the hole or turning or physically twisting of the handle of the driver (34) that engages the drive head so that the suture anchor (100, 200, 300) rotates by itself into the hole in bone.

2. The suture anchor (100, 200, 300) of claim 1, wherein the tip portion (20) tapers from the constant diameter of the central body (4) to a minimum diameter smaller than the constant diameter.

3. The suture anchor (100, 200, 300) of claim 1, further comprising two lengths of suture (32) attached to the suture anchor (100, 200, 300).

4. The suture anchor of claim 1, further comprising a driver (34) comprising a tubular shaft having a distal end and a proximal end, and a handle disposed on the proximal end of the shaft, wherein the suture anchor (100, 200, 300) is inserted into the distal end of the driver (34); and at least one strand of suture (32) is passed through the eyelet (12) in the suture anchor (100, 200, 300), through the tubular shaft of the driver (34), through at least a portion of the handle of the driver (34), and exiting the handle, the suture being secured to the handle so as to retain the suture anchor (100, 200, 300) on the distal end of the tubular shaft.

## Patentansprüche

1. Eingeschlagener Nahtanker (100, 200, 300), umfassend:
einen nicht-kanülierten zentralen Körper (4) mit einem distalen Ende (16) und einem proximalen Ende (8);
einem an dem distalen Ende (16) des zentralen Körpers (4) angeordneten Spitzenabschnitt (20), wobei der Spitzenabschnitt (20) eine verjüngte konische Gestalt aufweist, die in einer abgerundeten Spitze endet oder stumpf ist;
einen an dem proximalen Ende (8) des zentralen Körpers (4) geformten Treiberkopf (10), wobei der Treiberkopf (10) mit zwei Kanälen ausgestattet ist, die entlang Seiten des Treiberkopfs (10) zur Unterbringung von zumindest einem Strang eines Nahtmaterials (32) geformt sind;
eine Öse (12) zur Aufnahme des zumindest einen Strangs eines Nahtmaterials (32), die auf dem proximalen Ende (8) des zentralen Körpers (4) geformt ist; und **dadurch gekennzeichnet, dass** der zentrale Körper (4) einen konstanten Durchmesser aufweist und dass ein kontinuierliches Schraubengewinde eine Vierer-Gewindesteigung mit vier Schraubengewinden und einer 1-Gewindesteigung oder einer 2-Gewindesteigung umfasst, worin die Schraubengewinde in einer Spirale um eine gesamte Länge des zentralen Körpers (4) und um eine gesamte Länge des Spitzenabschnitts (20) angeordnet sind, worin das kontinuierliche Schraubengewinde ausgelegt ist, den Anker (100, 200, 300) zu veranlassen, sich bei Einführung in ein Loch in Knochen von selbst um ungefähr 60 bis ungefähr 100 Grad zu drehen, indem nur auf einen Griff eines Eintreibers (34), der in den Treiberkopf des Ankers (100, 200, 300) eingreift, gehämmert wird und der Eintreiber (34) und der Anker (100, 200, 300) in das Loch im Knochen geschlagen werden, ohne dass Gewindebohren des Lochs oder Drehen oder physikalisches Verdrehen des Griffs des mit dem Treiberkopf in Eingriff stehenden Eintreibers (34) notwendig ist, so dass sich der Nahtanker (100, 200, 300) von selbst in das Loch im Knochen dreht.

2. Nahtanker (100, 200, 300) nach Anspruch 1, worin sich der Spitzenabschnitt (20) von dem konstanten Durchmesser des zentralen Körpers (4) zu einem Mindestdurchmesser, der kleiner als der konstante Durchmesser ist, verjüngt.

3. Nahtanker (100, 200, 300) nach Anspruch 1, ferner zwei Längen des Nahtmaterials (32) umfassend, die an dem Nahtanker (100, 200, 300) befestigt sind.

4. Nahtanker nach Anspruch 1, ferner einen Eintreiber (34) umfassend, der einen röhrenförmigen Schaft mit einem distalen Ende und einem proximalen Ende, und einen auf dem proximalen Ende des Schafts angeordneten Griff umfasst, worin der Nahtanker (100, 200, 300) in das distale Ende des Eintreibers (34) eingeführt wird und zumindest ein Strang des Nahtmaterials (32) durch die Öse (12) in dem Nahtanker (100, 200, 300), durch den röhrenförmigen Schaft des Eintreibers (34), durch zumindest einen Abschnitt des Griffs des Eintreibers (34) geführt wird und aus dem Griff austritt, wobei das Nahtmaterial an dem Griff fixiert wird, um den Nahtanker (100, 200, 300) auf dem distalen Ende des röhrenförmigen Schafts zu halten.

## Revendications

1. Ancrage de suture à enfoncer (100, 200, 300) comprenant :
un corps central (4) ne présentant pas de canule et ayant une extrémité distale (16) et une extrémité proximale (8) ;
une portion de pointe (20) située au niveau de l'extrémité distale (16) du corps central (4), la portion de pointe (20) ayant une forme conique effilée se terminant par une pointe arrondie ou étant émoussée ;
une tête d'entraînement (10) formée sur l'extrémité proximale (8) du corps central (4), la tête d'entraînement (10) étant pourvue de deux canaux formés le long de côtés de la tête d'entraînement (10) pour recevoir au moins un fil de suture (32) ;
un oeillet (12) pour recevoir l'au moins un fil de suture (32) formé sur l'extrémité proximale (8) du corps central (4) ; et **caractérisé en ce que** le corps central (4) présente un diamètre constant et **en ce qu'**un filetage hélicoïdal continu comprend un filetage à quatre départs avec quatre filets hélicoïdaux et un pas de 1 filet ou un pas de 2 filets, les filets hélicoïdaux étant disposés en spirale autour d'une longueur totale du corps central (4) et autour d'une longueur totale de la portion de pointe (20), le filetage hélicoïdal continu étant configuré de manière à entraîner la rotation automatique de l'ancrage (100, 200, 300) de lui-même autour de 60 à environ 100 degrés lors de l'insertion dans un trou dans un os en tapant simplement sur une poignée d'un dispositif d'entraînement (34) s'engageant avec la tête d'entraînement de l'ancrage (100, 200, 300) et en enfonçant le dispositif d'entraînement (34) et l'ancrage (100, 200, 300) dans le trou dans l'os, et sans nécessiter le taraudage du trou ou son tournage ou la torsion physique de la poignée du dispositif d'entraînement (34) qui s'engage avec la tête d'entraînement de telle sorte que l'ancrage de suture (100, 200, 300) tourne de lui-même dans le trou dans l'os.

2. Ancrage de suture (100, 200, 300) selon la revendication 1, dans lequel la portion de pointe (20) est effilée depuis le diamètre constant du corps central (4) jusqu'à un diamètre minimum inférieur au diamètre constant.

3. Ancrage de suture (100, 200, 300) selon la revendication 1, comprenant en outre deux longueurs de suture (32) attachées à l'ancrage de suture (100, 200, 300).

4. Ancrage de suture selon la revendication 1, comprenant en outre un dispositif d'entraînement (34) comprenant une tige tubulaire ayant une extrémité distale et une extrémité proximale, et une poignée disposée sur l'extrémité proximale de la tige, l'ancrage de suture (100, 200, 300) étant inséré dans l'extrémité distale du dispositif d'entraînement (34) ; et au moins un fil de suture (32) étant passé à travers l'oeillet (12) dans l'ancrage de suture (100, 200, 300), à travers la tige tubulaire du dispositif d'entraînement (34), à travers au moins une portion de la poignée du dispositif d'entraînement (34), et sortant de la poignée, la suture étant fixée à la poignée de manière à retenir l'ancrage de suture (100, 200, 300) sur l'extrémité distale de la tige tubulaire.
